(19) Europäisches Patentamt
European Patent Office
Office européen des brevets

(11) Publication number: **0 024 907 A1**

(12) **EUROPEAN PATENT APPLICATION**

(21) Application number: 80302934.7

(22) Date of filing: 22.08.80

(51) Int. Cl.³: A 01 N 43/40
C 07 D 213/64, C 07 D 413/12

(30) Priority: 23.08.79 PC T/US79/00648
18.01.80 PC T/US80/00041

(43) Date of publication of application:
11.03.81 Bulletin 81/10

(84) Designated Contracting States:
BE GB IT NL SE

(71) Applicant: E.I. DU PONT DE NEMOURS AND COMPANY
Legal Department 1007 Market Street
Wilmington, Delaware 19898(US)

(72) Inventor: Ting, James J-S.
6616 Van Nuys Boulevard
Van Nuys California 91405(US)

(74) Representative: Hildyard, Edward Martin et al,
Frank B. Dehn & Co. Imperial House 15-19 Kingsway
London WC2B 6UZ(GB)

(54) **Pyridyloxyphenoxycrotonic acid derivatives, their preparation, compositions thereof and their use as selective weed control agents.**

(57) Compounds of the formula

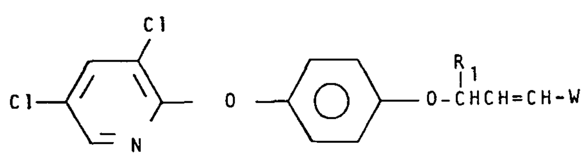

where

$R_1$ is H, $-CH_3$, $-CH_2CH_3$ or $-CH_2OCH_3$;

W is $COOR_2$, $CONR_3R_4$, $COSR_5$, COCl, CN or

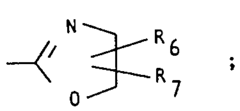

;

$R_2$ is H, $C_1$-$C_4$ alkyl, $-CH_2CH_2OCH_3$, $-CH_2CH_2OCH_2CH_3$ or $-CH_2CH_2CH_2OCH_3$;

$R_3$ is H, $C_1$-$C_3$ alkyl or $-OCH_3$;

$R_4$ is H or $C_1$-$C_3$ alkyl;

$R_5$ is $C_1$-$C_4$ alkyl; and

$R_6$ and $R_7$ are independently H or $CH_3$;

and agriculturally suitable salts thereof, exhibit herbicidal activity. They are of particular interest for selectively controlling grass weeds in broadleaf crops. For such use they can be formulated into compositions in conventional manner.

The novel compounds can be made by e.g. reacting 4-(3,5-dichloropyridin-2-yloxy)phenol with

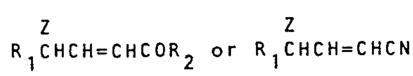

and if necessary transforming the product initially obtained.

EP 0 024 907 A1

0024907

"Pyridyloxyphenoxycrotonic acid derivatives, their preparation, compositions thereof and their use as selective weed control agents"

## Technical Field

This invention relates to pyridyloxyphenoxycrotonate esters and related compounds which are useful as selective weed control agents. The compounds are especially useful for controlling grass weeds in broadleaf crops such as soybeans.

The presence of undesirable vegetation causes substantial damage to useful crops, especially agricultural products that satisfy man's basic food and fiber needs, such as cotton, rice, corn, wheat, soybean and the like. The current population explosion and concomitant world food and fiber shortage underlie the need for improvements in the efficiency of producing these crops. Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

Background Art

Belgian Patent No. 875,880, dated August 16, 1979, to Ishihara Sangyo Kaisha, Ltd., claims herbicidal compounds of the formula:

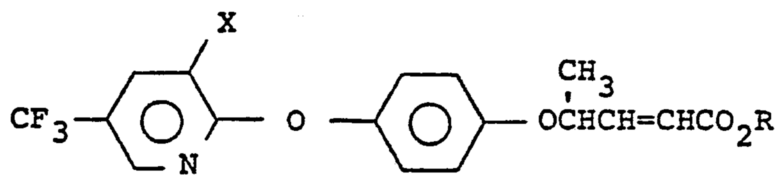

where

X is hydrogen or halogen and

R is alkyl of one to four carbon atoms.

Belgian Patent 871,523 to Kumiai, dated October 31, 1977, claims diphenyl ether herbicides. The following compound is representative of this disclosure:

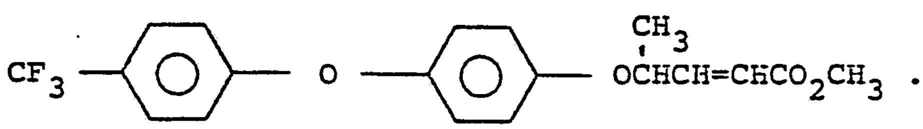

German Patent 2,639,796, assigned to Rohm and Haas and dated September 3, 1975, claims diphenyl ether herbicides of the type represented by the following formula:

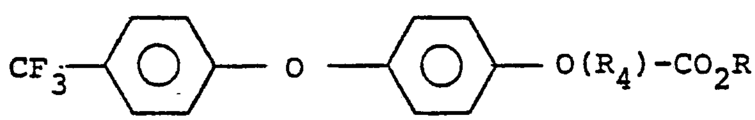

where

$R_4$ is alkylene or alkenyl of up to 5 carbon atoms.

Unexamined Japanese Patent 3,130,672, assigned to Ishihara Industries KK and dated April 18, 1977, claims phenoxy valeric acid derivatives such as

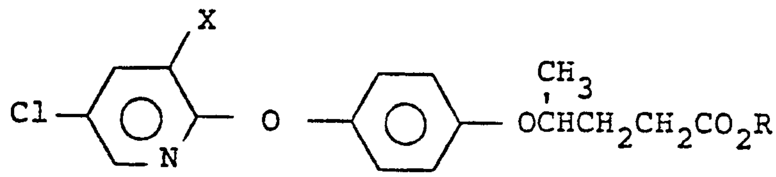

where

X = H or Cl.

German Patent 2,812,571, assigned to Ishihara Sangyo and dated July 21, 1977, claims phenoxy pyridine derivatives of the type represented by the formula:

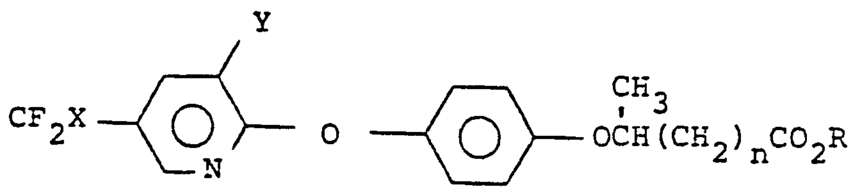

where

X = F or Cl,

Y = H or Cl, and

n = 0, 1 or 2.

## Summary of the Invention

This invention relates to novel compounds of Formula I, to their preparation, agricultural compositions containing them and to their method of use as pre- and post-emergence herbicides.

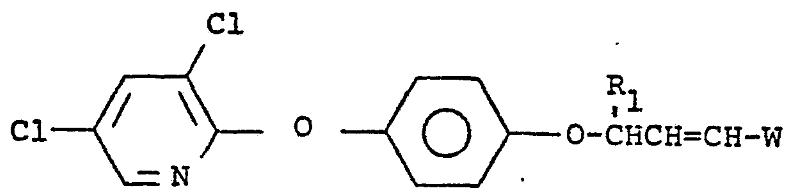

I

where

$R_1$ is H, $-CH_3$, $-CH_2CH_3$ or $-CH_2OCH_3$;

W is $COOR_2$, $CONR_3R_4$, $COSR_5$, COCl, CN or

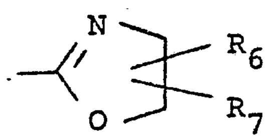

;

$R_2$ is H, $C_1$-$C_4$ alkyl, $-CH_2CH_2OCH_3$, $-CH_2CH_2OCH_2CH_3$ or $-CH_2CH_2CH_2OCH_3$;

$R_3$ is H, $C_1$-$C_3$ alkyl or $-OCH_3$;

$R_4$ is H or $C_1$-$C_3$ alkyl;

$R_5$ is $C_1$-$C_4$ alkyl; and

$R_6$ and $R_7$ are independently H or $CH_3$;

provided that when $R_3$ is $-OCH_3$ then $R_4$ is $CH_3$.

This invention also relates to agriculturally suitable salts of the above described compound where

W is $COOR_2$ and

$R_2$ is hydrogen.

The preferred salts are sodium or potassium salt.

## Detailed Description of the Invention

### Preferred Compounds

Preferred for their high activity and/or favorable cost are those compounds of Formula I wherein independently:

$R_1$ is $CH_3$; and

W is $\overset{\overset{O}{\|}}{C}OR_2$ or CN.

More preferred for their higher activity and/or even more favorable cost are those preferred compounds wherein

W = $COOR_2$; and

$R_2$ = H, $CH_3$ or $C_2H_5$.

Most preferred for their even higher activity and/or even more favorable cost are those more preferred compounds where

$R_2$ = H or $CH_3$.

Specifically preferred for high activity and favorable cost are:

4-[4-(3,5-dichloropyridin-2-yloxy]phenoxy]-2-butenenitrile;

4-[4-(3,5-dichloropyridin-2-yloxy)phenoxy]-2-pentenenitrile;

Methyl 4-[4-(3,5-dichloropyridin-2-yloxy)phenoxy]-2-pentenoate; and

4-[4-(3,5-dichloropyridin-2-yloxy)phenoxy]-2-pentenoic acid.

The D-enantiomer is believed to be about twice as active as the racemate.

Methods of Preparation

The compounds of Formula I, in which W is $\overset{O}{\overset{\|}{C}}OR_2$ or CN can be prepared, as shown in Equation A, by the reaction of 4-(3,5-dichloropyridin-2-yloxy)-phenol (Formula II),

Equation A

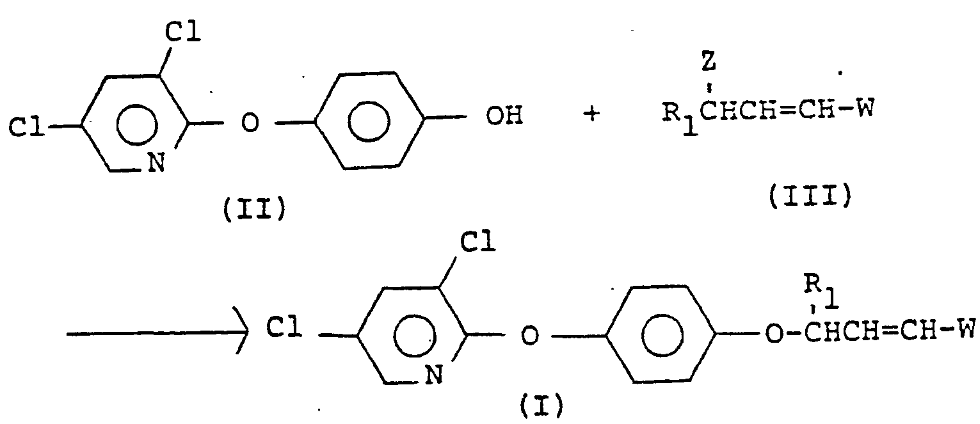

(II) (III) (I)

x

and a substituted alkenoic acid derivative of the Formula III, wherein $R_1$ is as defined hereinbefore and wherein Z is Cl or Br. An approximately equimolar amount of the compound of Formula II and the compound of Formula III are contacted in the presence of a base. Suitable bases include an alkali metal or alkali earth metal hydroxide, hydride, carbonate, bicarbonate or alkoxide, for example, sodium methoxide or potassium _t_-butoxide. The base is present in an amount of about 1 to 2 molar times the amount of the compound of Formula II. The reaction can be carried out in an organic solvent such as methylethyl ketone, tetrahydrofuran, dimethylformamide, dimethylsulfoxide, or dioxane at temperatures about 20°-100°C, preferably about 25-80°C. In this reaction procedure (and the reaction procedures relating to compounds IV, V, VI, described below) pressure is not critical since pressures above and below atmospheric are suitable. For convenience, atmospheric pressure is preferred.

The compounds of Formula II are known in the art, for example, in the teachings of U.S. 4,046,553 and Belgian Patent 865,136, the disclosures of which are hereby incorporated by reference. The substituted alkenoic acid derivatives of Formula III are prepared according to the teachings of _Justis Liebigs Annalen Der. Chemie_ _576_, 155 (1952) and _Otdel, Khim. Nauk._ 1512 (1960), also hereby incorporated by reference.

The compounds of Formula IV,

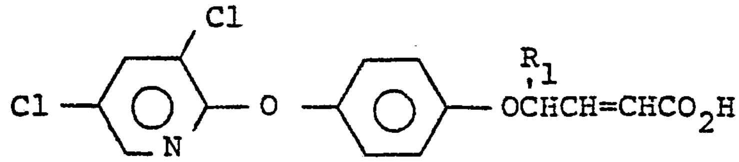

(IV)

where $R_1$ is as defined hereinbefore, can be prepared by the acidic hydrolysis of the compounds of Formula I with hydrochloric acid or sulfuric acid, or by the basic hydrolysis of the compounds of Formula (I) with sodium hydroxide, potassium hydroxide, or barium hydroxide followed by acidification with hydrochloric acid or sulfuric acid. The preparation of the compounds of Formula IV can be carried out in a solvent such as a mixture of methanol and water, or of ethanol and water at temperatures of about 25-100°C.

The compounds of Formula IVa,

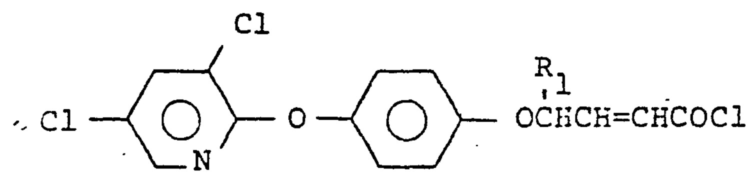

IVa

in which $R_1$ is as defined hereinbefore, can be prepared by treatment of the appropriate carboxylic acid (IV) with an excess of oxalyl chloride or thionyl chloride.

The compounds of Formula V,

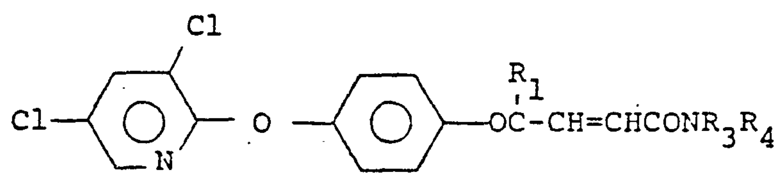

(V)

in which W is $\overset{O}{\overset{\|}{C}}NR_3R_4$ and where $R_1$, $R_3$ and $R_4$ are as defined hereinbefore, can be prepared by contacting the above acid chloride IVa with the proper amine. Typically, the amine is present in an amount of about two molar times the amount of acid chloride. The reaction can be carried out neat or in an organic solvent such as methylene chloride, chloroform, tetrahydrofuran, or dioxane at ambient temperature. The acid chloride can be prepared by reacting the compounds of Formula IV with thionyl chloride, a method known in the art.

The compounds of Formula VI,

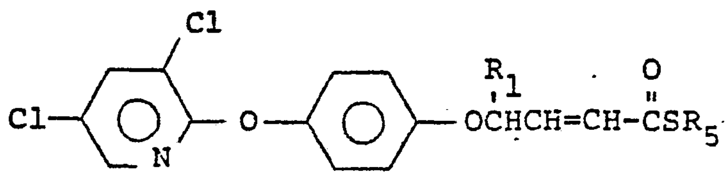

(VI)

in which W is $\overset{O}{\overset{\|}{C}}SR_5$ and where $R_1$ and $R_5$ are as defined hereinbefore, can be obtained by reacting the corresponding acid chloride of the Formula IVa above with the proper mercaptan in the presence of a base. The acid chloride, the compound of Formula IV and the base are typically present in approximately equimolar proportions. The reaction can be carried out in an organic solvent such as methylene chloride, tetrahydrofuran or toluene. The reaction can be carried out at temperatures between about 0° and 100°C, preferably between about 25° and 50°C. The base used for the preparation of the compounds of Formula VI can be a tertiary organic amine such as trimethylamine, triethylamine, N,N-dimethylaniline or pyridine, or alkali metal or alkali earth metal carbonate or bicarbonate.

Agriculturally suitable salts of compounds of Formula I are also useful herbicides and can be prepared by a number of ways known to the art. For example, metal salts can be made by treating compounds of Formula I wherein W is $-\overset{\overset{O}{\|}}{C}OR_2$ and $R_2$ is hydrogen with a solution of an alkali or alkaline earth metal salt having a sufficiently basic anion (e.g. hydroxide, alkoxide, carbonate or hydride). Ammonium, amine, and quaternary amine salts can be made by similar techniques.

Salts of compounds of Formula I wherein W is $\overset{\overset{O}{\|}}{C}OR_2$ and $R_2$ is hydrogen can also be prepared by exchange of one cation for another. Cationic exchange can be effected by direct treatment of an aqueous solution of a salt of a compound of Formula I (e.g. alkali metal or quaternary ammonium salt) with a solution containing the cation to be exchanged. This method is most effective when the desired salt containing the exchanged cation is insoluble in water and can be separated by filtration.

Exchange may also be effected by passing an aqueous solution of salt of a compound of Formula I (e.g. an alkali metal or quaternary ammonium salt) through a column packed with a cation exchange resin containing the cation to be exchanged. In this method, the cation of the resin is exchanged for that of the original salt and the desired product is eluted from the column. This method is particularly useful when the desired salt is water soluble.

The compounds of Formula VII,

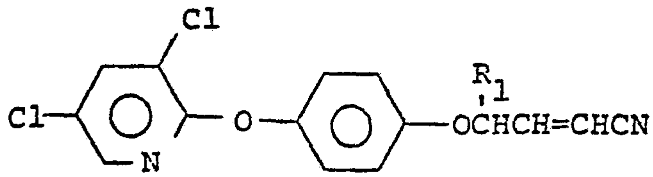

(VII)

in which W is CN and $R_1$ is defined as hereinbefore, can be prepared by the reaction of the phenol of Formula II, and a substituted alkenenitrile derivative of the Formula III.

The compounds of Formula VIII,

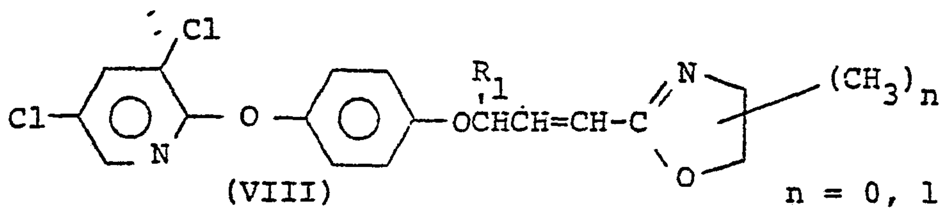

(VIII)

n = 0, 1, 2

in which $R_1$ is defined as hereinbefore, can be prepared by an acid chloride of Formula IVa with an appropriately substituted β-aminoethanol. The resulting amide is cyclized in thionyl chloride to give the isoxazolines, represented by Formula VIII.

The D-enantiomer, which is believed to be about

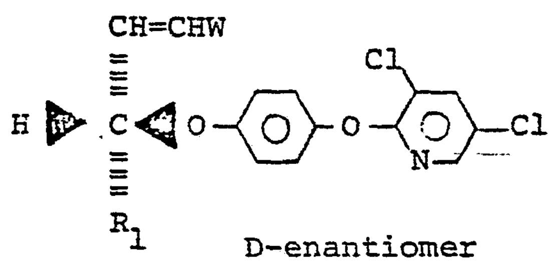

D-enantiomer stereoprojection

twice as active as the racemate, can be prepared by the reaction of a substituted phenol of the Formula II and the optically active (ℓ-configuration) alkenoic acid derivative or by resolution of the D,L-carboxylic acid IV ($R_1$≠H) by crystallization of salts formed with optically active bases. Both methods are well known in the art.

In the following examples, all parts and percentages are by weight and temperatures are in degrees centigrade unless otherwise specified.

Example I

Methyl 4-[4-(3,5-dichloropyridin-2-yloxy)phenoxy]-2-pentenoate

To 2.5 g (0.0098 mole) of 4-(3,5-dichloro-2-pyridinyloxy)phenol, and 1.5 g of potassium carbonate in 40 ml of methyl ethyl ketone, 2.5 g of methyl 4-bromo-2-pentenoate, dissolved in 10 ml of methyl ethyl ketone, was added at room temperature with constant stirring. The mixture was heated at reflux for 20 hours. Upon cooling to room temperature, the mixture was filtered through celite and the insoluble materials washed with 50 ml of ethyl ether. An oily residue which remained after concentrating the filtrate under reduced pressure was dissolved in 125 ml of ethyl ether and washed with excess water. Drying over magnesium sulfate, charcoal filtering, and removal of solvent and volatile impurities at reduced pressure (0.35 mm Hg, 1.5 hours at 75°) gave 2.5 g (60%) of product, $N_D^{25}$ 1.5790.

IR (neat): 5.75 ($CO_2CH_3$), 5.95, 6.30 (C=C), 6.60, 6.90, 11.95 microns; NMR ($CDCl_3$): δ 1.50 (d, 3H, $CH_3$, J=7 Hz), 3.75 (s, 3H, $OCH_3$), 4.75-5.15 (m, 1H, allylic hydrogen), 5.85-6.20 (m, 1H, vinylic proton), 6.70-7.20 (m, 5H, vinylic proton and phenol protons), 7.60-7.90 (m, 2H, pyridine protons).

## Example II

**Ethyl 4-[4-(3,5-dichloropyridin-2-yloxy)phenoxy]-2-butenoate**

To 5.1 g (0.02 mole) of 4-(3,5-dichloro-2-pyridinyloxy)phenol in 40 ml of methyl ethyl ketone is added 5.1 g of ethyl 4-bromo-2-butenoate, dissolved in 10 ml of methyl ethyl ketone, followed by the addition of 2.8 g anhydrous potassium carbonate. The mixture was refluxed 18 hours with continuous stirring. After evaporation of methyl ethyl ketone under reduced pressure, the residue was washed with water, extracted into ethyl ether, dried over magnesium sulfate, and concentrated. Purification by dry column chromatography in ethyl ether/hexane (1:1) gave 0.6 g (8%) of product.

IR (neat): 5.75 ($CO_2C_2H_5$) microns; NMR ($CDCl_3$): δ 1.30 (t, 3H, $CH_3$), 4.30 (q, 2H, $OCH_2$), 4.80 (m, 2H, allylic protons); 8.40 (m, 2H, vinylic protons), 7.0-8.0 (m, 6H, aromatic protons).

Following the teaching of Examples I and II, and by reacting 4-(3,5-dichloropyridin-2-yloxy)-phenol and an appropriate haloalkenoate, the compounds in Table 1 can be prepared.

Table 1

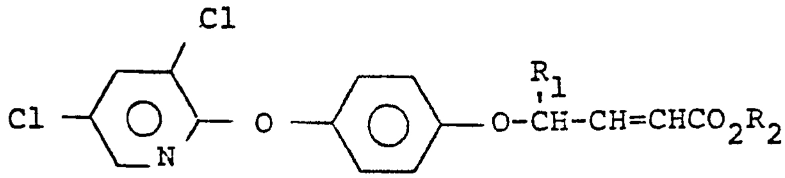

| $R_1$ | $R_2$ | $N_D^{25}$ |
|---|---|---|
| H | $C_2H_5$ | 1.5606 |
| $CH_3$ | $CH_3$ | 1.5790 |
| $CH_3$ | $-(CH_2)_3CH_3$ | |
| $CH_3$ | $-CH(CH_3)C_2H_5$ | |
| $CH_3$ | $-CH_2CH(CH_3)_2$ | |
| $C_2H_5$ | $-CH_3$ | 1.5628 |
| $-CH_2OCH_3$ | $-CH(CH_3)_2$ | |
| $CH_3$ | $-CH_2CH_2OC_2H_5$ | |
| $CH_3$ | $-C_2H_5$ | 1.5589 |
| $CH_3$ | $-CH(CH_3)_2$ | |
| $CH_3$ | $-C(CH_3)_3$ | |
| $CH_3$ | $-CH_2CH_2CH_3$ | |
| $CH_3$ | $-CH_2CH_2OCH_3$ | |
| H | $CH_3$ | |
| $C_2H_5$ | $CH_2CH_2CH_2OCH_3$ | |

## Example III

4-[4-(3,5-dichloropyridin-2-yloxy)phenoxy]-2-pentenoic acid

To 13.0 grams of methyl-4-[4-(3,5-dichloro pyridin-2-yloxy)phenoxy]-2-pentenoate in a 250 ml Evlenmeyer flask, 11.0 grams of 50% sodium hydroxide was added, followed by the addition of 50 ml of methanol and 10 ml of distilled water. Initially, the mixture was warmed on a steam bath for 5 minutes to form a solution which was then stirred at room temperature for 2.5 hours. Excess water (150 ml) was added and the solution neutralized with glacial acetic acid. The oily solid, which precipitated immediately, was extracted into 200 ml of ethyl acetate and separated. After washing the organic phase with brine, drying ($MgSO_4$), and filtering, the solvent was evaporated to yield an oily residue which was crystallized from 1-chlorobutane, yield 5.5 g, m.p. 143-145°.

NMR ($CDCl_3$):$\delta$ 1.35 (d, 3H, $CH_3$);
4.50-5.00 (m, 1H, allylic hydrogen);
5.70-6.15 (m's, vinylic proton);
6.60-7.45,
7.60-7.90 (m's, ArH and vinylic proton).

IR (Nujol): 5.85 (c=o), 12.00 (broad) microns.

By hydrolysis of an appropriate ester employing the procedure of Example III, the carboxylic acids in Table 2 can be prepared.

Table 2

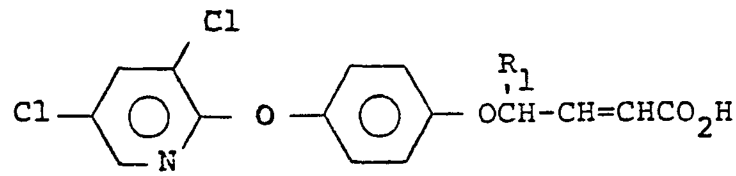

| $R_1$ | m.p. (°C) |
|---|---|
| H | |
| $CH_3$ | 143-145° |
| $C_2H_5$ | |
| $CH_2OCH_3$ | |

Example IV

Sodium 4-[4-(3,5-dichloropyridin-2-yloxy)phenoxy]-2-pentenoate

A mixture of 1.2 grams 4-[4-(3,5-dichloropyridin-2-yloxy)phenoxy]-2-pentenoic acid and 0.2 grams powdered sodium hydroxide in 5 ml of methanol in a 50 ml flask was warmed on a steam bath for 10 minutes. After cooling to room temperature, excess ethyl ether was added with stirring and the insoluble product filtered, washed with ether and dried, yield 0.9 grams, m.p. 238-245° (dec.).

NMR (tfa-D): $\delta$ 1.65 (d, 3H, $CH_3$);
4.95-5.20 (m, 1H, allylic hydrogen);
6.05-6.40 (m's, 1H, vinylic proton);
7.10-7.60,
8.15-8.65 (m's, ArH and vinylic proton).

IR (Nujol): 6.0 (c=c), 12.00 (broad) microns.

x

## Example V

**Potassium 4-[4-(3,5-dichloropyridin-2-yloxy)phenoxy]-2-pentenoate**

In a 50 ml flask, a solution of 1.2 grams of 4-[4-(3,5-dichloropyridin-2-yloxy)phenoxy]-2-pentenoic acid and 0.3 grams powdered potassium hydroxide in 5 ml of methanol was warmed on a steam bath for 10 minutes. Under reduced pressure, the solvent was removed and the remaining solid suspended in ethyl ether and filtered, yield 1.0 grams, m.p. 217-226° (dec.).

NMR (tfa-D):δ 1.65 (d, 3H, $CH_3$);
4.95-5.20 (m, 1H, allylic hydrogen);
6.00-6.40 (m's, 1H, vinylic proton);
6.95-7.60,
8.10-8.60 (m's, ArH and vinylic proton).

IR (Nujol): 6.00 (c=c), 12.00 (broad) microns.

By treatment of the appropriate carboxylic acid with a suitable base such as ammonia, an amine, quaternary ammonium hydroxide, or alkali metal or alkaline earth hydroxide, hydride, carbonate, or bicarbonate, the following carboxylic acid salts may be prepared by the methods of Examples IV and V.

Table 3

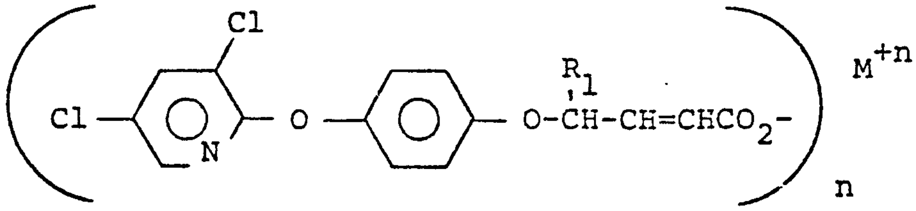

| $R_1$ | n | M | m.p. (°C) |
|---|---|---|---|
| H | 1 | Na | |
| $CH_3$ | 1 | Na | 238-245° (dec.) |
| $CH_3$ | 1 | K | 217-226° (dec.) |
| $CH_3$ | 1 | Li | |
| $CH_3$ | 1 | $(HOCH_2CH_2)_3NH$ | |
| $CH_3$ | 1 | $NH_4$ | |
| $CH_3$ | 1 | $CH_3NH_3$ | |
| $C_2H_5$ | 2 | Mg | |
| $CH_3$ | 2 | Ca | |
| $CH_2OCH_3$ | 2 | Ca | |
| $C_2H_5$ | 1 | $(HOCH_2CH_2)_2NH_2$ | |
| $CH_2OCH_3$ | 1 | $(CH_3)_2NH_2$ | |
| $CH_3$ | 1 | $HOCH_2CH_2NH_3$ | |
| $CH_3$ | 1 | $(CH_3)_3NH$ | |
| $CH_3$ | 1 | $(CH_3)_4N$ | |
| $CH_3$ | 1 | $(CH_3CH_2)_3NH$ | |
| $CH_3$ | 1 | $(HOCH_2CH_2)_2NH_2$ | |

Example VI

N-Methyl-4-[4-(3,5-dichloropyridin-2-yloxy)phenoxy]-2-pentenamide

The following procedure can be employed to make the title compound.

To a mixture of 2.0 grams 4-[4-(3,5-dichloropyridin-2-yloxy)phenoxy]-2-pentenoic acid and 7 ml of toluene stirring magnetically in a 100 ml RB flask, 2.0 ml of oxalyl chloride was added at 0°. Gradually, the stirred suspension was allowed to reach room temperature. After stirring overnight at room temperature, the initial suspension changed to a clear orange solution. Under reduced pressure, the toluene and excess oxalyl chloride was removed and the crude off white acid chloride remained. With cooling in an icebath, 25 ml of 40% methylamine solution was slowly added with occasional stirring by spatula. After stirring for 10 minutes, excess water was added and the oily brown insoluble solid was extracted into 150 ml of a ethyl acetate/ethyl ether mixture (1:1). The extract was washed twice with brine, dried ($MgSO_4$), filtered and evaporated to yield an oily residue which was crystallized from 1-chlorobutane, yield 1.0 gram, m.p. 147-149°.

NMR (tfa-D):δ 1.60 (d, 3H, $CH_3$);
3.20 (s, 3H, $CH_3$);
5.00-5.45 (m, 1H, allylic hydrogen);
6.25-6.75 (m's, 1H, vinylic proton);
7.00-7.50,
8.05-8.70 (m's, ArH and vinylic proton).

IR (Nujol): 3.0 (NH), 5.90 (c=o), 6.10 (c=c), 12.00 microns.

By treatment of an appropriate acid chloride with an appropriate amine, by the procedure of Example VI, the amides in Table 4 are prepared.

Table 4

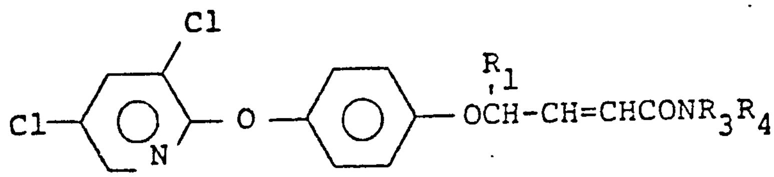

| $R_1$ | $R_3$ | $R_4$ | m.p.(°C) |
|---|---|---|---|
| H | H | H | |
| $CH_3$ | H | H | 148-151° |
| H | $CH_3$ | H | |
| $CH_3$ | $CH_3$ | H | 147-149° |
| $CH_3$ | $OCH_3$ | $CH_3$ | |
| $CH_3$ | $CH_3$ | $CH_3$ | |
| $CH_3$ | $CH_2CH_2CH_3$ | H | |
| $CH_3$ | $CH(CH_3)_2$ | H | |
| $C_2H_5$ | $CH_3$ | $C_2H_5$ | |
| $CH_2OCH_3$ | $CH_3$ | $CH(CH_3)_2$ | |
| $CH_3$ | $CH(CH_3)_2$ | $CH(CH_3)_2$ | |
| $CH_3$ | $C_2H_5$ | H | |
| $CH_3$ | $CH(CH_3)_2$ | H | |

Example VII

Methyl 4-[4-(3,5-dichloropyridin-2-yloxy)phenoxy]-2-pentenethioate

The following procedure can be employed to make the title compound.

To a mixture of 6.9 g of 4-[4-(3,5-dichloropyridin-2-yloxy)phenoxy]-2-pentenoyl chloride and 2.0 g of triethylamine in 30 ml of methylene chloride, cooled in an ice-acetone bath, add dropwise 1.1 ml of liquified methanethiol. Allow the reaction mixture to warm to room temperature, stir for 1 hour, and wash with water. Remove methylene chloride under vacuum to afford methyl 4-[4-(3,5-dichloropyridin-2-yloxy)phenoxy]-2-pentenethioate.

By treatment of an appropriate acid chloride with an appropirate mercaptan following the procedure of Example VII, the thiolesters listed in Table 5 can be obtained.

Table 5

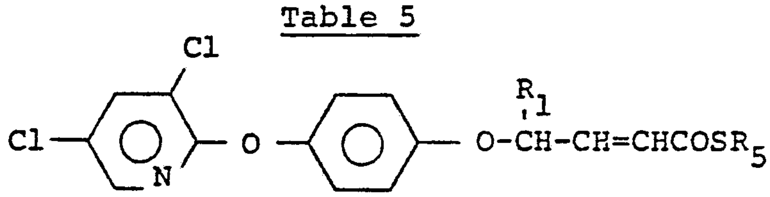

| $R_1$ | $R_5$ |
|---|---|
| H | $CH_3$ |
| $CH_3$ | $CH_3$ |
| $CH_3$ | $C_2H_5$ |
| $CH_3$ | $CH(CH_3)_2$ |
| $CH_3$ | $CH_2CH_2CH_2CH_3$ |
| $C_2H_5$ | $CH_3$ |
| $CH_2OCH_3$ | $C_2H_5$ |
| $CH_3$ | $CH(CH_3)C_2H_5$ |
| $CH_3$ | $CH_2CH(CH_3)_2$ |

Example VIII

4-[4-(3,5-dichloropyridin-2-yloxy)phenoxy]-2-pentenenitrile

In a 100 ml RB flask, a mixture of 3.5 grams 4-(3,5-dichloropyridin-2-yloxy)phenol, 3.5 grams potassium carbonate, and 3.5 grams of 4-bromo-2-pentenenitrile was stirred magnetically in 35 ml of dry acetonitrile at room temperature for 4 days. Ethyl ether (60 ml) was added to the mixture and the resulting dark suspension filtered and the insoluble material washed with ether. The volume of the filtrate was increased to 350 ml by adding ethyl ether and then washed twice with water, once with saturated sodium bicarbonate and once with brine. After drying over $MgSO_4$ and filtering, the solvent was evaporated to yield an oil, which was purified by silica gel column chromatography (methylene chloride as the eluent) and subsequent crystallization from hexane, yield 1.5 g, m.p. 93-96°.

NMR ($CDCl_3$): $\delta$ 1.45 (d, 3H, $CH_3$);
4.70-5.20 (m, 1H, allylic proton);
5.45-5.85 (m's, 1H, vinylic proton);
6.60-7.30,
7.70-8.00 (m's, ArH, and vinylic proton).

IR (Nujol): 4.44 (C≡N), 9.37, 11.95 (broad) microns.

## Example IX

4-[4-(3,5-dichloropyridin-2-yloxy)phenoxy]-2-butenenitrile

To a mixture of 1.9 grams 4-(3,5-dichloropyridin-2-yloxy)phenol, 3.0 grams of potassium carbonate and 40 ml of methyl ethyl ketone stirring magnetically in a 100 ml RB flask, 4.0 grams 4-bromo-2-butenenitrile was added and the mixture heated under reflux for 24 hours. Ethyl ether (150 ml) was added at room temperature and the black suspension filtered and the insoluble material washed with ether. After transferring to a separatory funnel, the filtrate was washed with saturated sodium carbonate and water, dried ($MgSO_4$), charcoal filtered and evaporated to yield a dark oil, which was purified by silica gel column chromatography (1-chlorobutane as the eluent), yield 1.5 g $N_D^{25}$ 1.5681.

IR (neat): 4.40 (C≡N), 6.60, 7.00, 8.35, 12.00 (broad) microns.

## Example X

Oxazole [3,5-dichloropyridin-2-yloxy(phenoxy)-2-pentenyl]-4,5-dihydro-4,4-dimethyl

To 0.6 grams of N-(1,1-dimethyl-2-hydroxyethyl)-4-[4-(3,5-dichloropyridin-2-yloxy)phenoxy]-2-pentenamide in a 50 ml flask, 1 ml of thionyl chloride was added and the mixture stirred at ambient temperature for 3.5 hours. Under reduced pressure, the excess thionyl chloride was removed and the remaining solid treated with saturated sodium bicarbonate, before extracting into 100 ml of ethyl ether. After washing with brine, the ether layer was dried ($MgSO_4$), filtered and evaporated to yield 0.4 g of crude isoxazoline.

IR (neat): 6.68 (C=N), 8.40, 9.40, 12.70 (broad) microns.

The compounds in Table 6 may be prepared by methods analogous to those used in the preceeding Examples VIII, IX and X.

Table 6

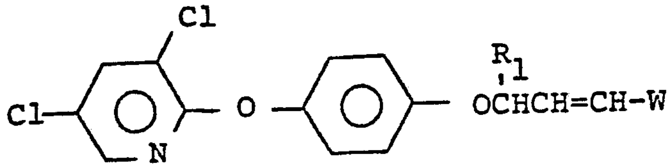

| $R_1$ | W | $N_D^{25}$ |
|---|---|---|
| H | CN | 1.5681 |
| $CH_3$ | CN | 1.5572 |
| $C_2H_5$ | CN | |
| H | 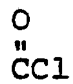 | |
| $CH_3$ | 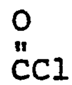 | |
| $CH_3$ | 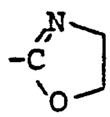 | |
| $CH_3$ | 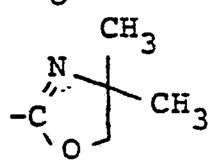 | |
| H | 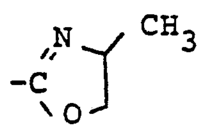 | |

Formulations

Useful formulations of the compounds of Formula I can be prepared in conventional ways. They include dusts, granules, pellets, solutions, suspensions, emulsions, wettable powders, emulsions, concentrates and the like. Many of these may be applied directly. Sprayable formulations can be extended in suitable media and used at spray volumes of from a few pints to several hundred gallons per acre. High-strength compositions are primarily used as intermediates for further formulation. The formulations, broadly, contain about 1% to 99% by weight of active ingredient(s) and at least one of a) about 0.1% to 20% surfactant(s) and b) about 1% to 99% solid or liquid diluent(s). More specifically, they will contain these ingredients in the following approximate proportions:

| | Percent by Weight | | |
|---|---|---|---|
| | Active Ingredient | Diluent(s) | Surfactant(s) |
| Wettable Powder | 20-90 | 0-74 | 1-10 |
| Oil Suspensions Emulsions, Solutions (including Emulsifiable Concentrates | 5-50 | 40-95 | 0-15 |
| Aqueous Suspensions | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd. Edn., Dorland Books, Caldwell, N.J. The more absorptive diluents are preferred for wettable powders and the denser ones for dusts. Typical liquid diluents and solvents are described in Marsden, "Solvent Guide", 2nd. Edn., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Co., Ridgewood, N.J., as well as Sisely and Wood, "Encyclopedia of Surface Active Agents", Chemical Publ. Co., Inc., New York, 1964, list surfactants and recommended uses. All formulations can contain minor amounts of additives to reduce foaming, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon performed granular carriers or by agglomeration techniques. See

J. E. Browning, "Agglomeration", Chemical Engineering, December 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knüsli, U.S. Patent 2,891,855, June 23, 1959, Col. 3, line 66 through Col. 5, line 17 and Examples 1-4;

G. C. Klingman, "Weed Control as a Science", John Wiley & Sons, Inc., New York, 1961, pp. 81-96;

J. D. Fryer and S. A. Evans, "Weed Control Handbook", 5th Edn., Blackwell Scientific Publications, Oxford, 1968, pp. 101-103.

A. Wettable Powder

| | |
|---|---|
| Methyl 4-[4-(3,5-dichloropyridin-2-yloxy)-phenoxy]-2-pentenoate | 35% |
| hydrous calcium silicate | 61% |
| sodium ligninsulfonate | 3% |
| sodium dioctylsulfosuccinate | 1% |

The ingredients are thoroughly blended, passed through a hammer mill and sifted through a U.S.S. No. 50 sieve (0.3 mm openings) before packaging.

All compounds of the invention can be formulated in similar manner.

B. Solution

| | |
|---|---|
| Methyl 4-[4-(3,5-dichloropyridin-2-yloxy)-phenoxy]-2-pentenoate | 50% |
| dimethylformamide | 50% |

The ingredients are stirred together to make a solution for direct low-volume application.

C. Granule

| | |
|---|---|
| Solution of Example B | 10% |
| attapulgite granules (0.71/0.30 mm) | 90% |

The solution is sprayed upon the performed clay granules in a double-cone blender.

D. Emulsifiable Concentrates

| | |
|---|---|
| Methyl 4-[4-(3,5-dichloropyridin-2-yloxy)-phenoxy]-2-pentenoate | 30% |
| blend of oil soluble sulfonates and polyoxyethylene ethers | 6% |
| cyclohexanone | 64% |

The ingredients are combined and stirred with gentle warming to speed mixing. A fine-screen filter is included in the packaging operation to remove any extraneous undissolved material.

Compositions can contain, in addition to the active ingredients of this invention, other conventional agricultural chemicals such as fertilizers, plant growth modifiers or herbicides.

For example, the compounds of Formula I can be combined with the following herbicides:

(1) 5-tert-butyl-3-(2,4-dichloro-5-isopropoxyphenyl)-1,3,4-oxadiazol-2-one;

(2) 6-methylthio-2,4-bis(ethylamino)-s-triazine;

(3) 3-isopropyl-(1H)-benzo-2,1,3-thidiazin-4-one 2,2-dioxide;

(4) 2,4-dichlorophenoxyacetic acid and related esters and salts;

(5) S-(2,2,3-trichloroallyl)-diisopropylthiocarbamate;

(6) Methyl 2-[4-(2,4-dichlorophenoxy(phenoxy)]-propanoate;

(7) 1,2-dimethyl-3,5-diphenyl-1H-pyrazolium methyl sulfate; and

(8) 4-chloro-2-butynyl 3-chlorocarbanilate.

The compounds of Formula I can also be combined with other herbicides and are particularly useful in combination with bromacil [3-(sec-butyl)-5-bromo-6-methyluracil], diuron [3,(3,4-dichlorophenyl)-1,1-dimethylurea], 3-cyclohexyl-1-methyl-6-dimethylamino-s-triazine-2,4(1H,3H)-dione, paraquat [1,1'-dimethyl-4,4'-bipyridinum ion], m-(3,3-dimethylureido)-phenyl tert-butylcarbamate, 2-methyl-4-chlorophenoxyacetic acid, its salts or esters, 4-amino-6-tert-butyl-3-methylthio-as-thio-as-triazine-5(4H)-one bentazone [3-isopropyl-1H-2,1,3-benzothiadiazin-(4)-3H-one-2,2-dioxide, and linuron [3-(3,4-dichlorophenyl)-1-methoxy-1-methyl urea] for controlling a broad spectrum of weeds.

The agricultural chemicals listed above are examplary of the compounds which can be mixed with the active compounds and are not intended to limit the invention in any way.

Use

The compounds of the present invention are useful when applied as pre- and/or post-emergence treatments for broad-spectrum control of a wide variety of weed and brush species growing on industrial sites, storage lots, along fences and building foundations, along railroad and utility rights-of-way, etc. In addition, the compounds of the invention have utility for weed control in certain crops, such as soybean, cotton, sugarbeets and beans.

These herbicides are particularly useful for selectively removing and controlling grass weeds, including volunteer corn, in broadleaf crops including soybeans, cotton, sugarbeets, beans, flax, cabbage, tomatoes, potatoes, peanuts, carrots, cucurbits, endive, beets, etc. Grassweeds include crabgrass, (Digitaria spp.), foxtail (Setaria spp.), barnyardgrass (Echinochloa crusgalli), wild oats (Avena fatua), johnsongrass (Sorghum halepense), fall panicum (Leptoloma cognatum), cheatgrass (Bromus tectorum), quackgrass (Agropyron repens), and orchardgrass (Dactylis glomerata). Compounds of this invention show a remarkable and unexpected degree of safety to broadleaf crops and an unusual phytotoxicity to grass weeds whether applied to the soil before weeds and crops emerge, that is, preemergence or whether applied postemergence including spraying on the weeds and crops. In addition, the compounds are useful when applied as preemergence or postemergence treatments alone and in combination with

other herbicides or surfactants for broad-spectrum control of a wide variety of weeds and brush species growing on industrial sites, on storage lots and along fences, building foundations, railroad, highway and utility rights-of-way etc.

Compounds of the present invention are considered to show an unexpected degree of safety on broadleaf crops as demonstrated by use of methyl 4-[4-(3,5-dichloropyridin-2-yloxy)phenoxy]-2-pentenoate on soybean, cotton and beans. Compounds of the present invention are also considered to show an unexpected degree of safety on wheat. The compound methyl 4-[4-(3,5-dichloropyridin-2-yloxy)phenoxy]-2-pentenoate has been shown to selectively control weeds in wheat while the closely related compound methyl 4-[4-(3-trifluoromethylpyridin-2-yloxy)phenoxy]-2-pentenoate caused severe wheat injury.

The precise amount of the compounds of this invention to be used in any particular situation will vary widely according to the end result desired. Factors effecting the optimum rate of application include the plant species to be controlled, soil type, formulation used, prevailing weather conditions, foliage density, length of time for which residual activity is desired, etc. Broadly speaking, the compounds are used at levels at about 0.06 to 20 kilograms per hectare, preferably approximately 0.125 to 5 kilograms per hectare. In general, the higher rates of application from within this range will be selected for adverse conditions or where extended persistence in soil is desired, and the lower rates for weed control in crops.

The herbicides effectively control grass weeds as demonstrated by the examples, but they do not control broadleaf weeds at low application rates. To obtain control of a wider spectrum of both broadleaf and grass weeds, combination treatments consisting of compounds of this invention with other herbicides effective on broadleaf weeds may be used to advantage. Combination treatments may be used with the components applied simultaneously as in a tank mix or mixed formulation, or sequentially with either or both components applied preplant incorporated, preemergence, postemergence, postemergence-directed, broadcast, band or spot treatment or any combination of these methods. The following examples of combination utility are cited:

Compounds of this invention with -

| Other Herbicide | Use |
|---|---|
| bentazon (post-) | Soybeans |
| 2,4-DB (post-) | Peanuts, Soybeans Alfalfa, Clover |
| Simazin (pre-) | Nursery, Citrus, Peaches, Established Alfalfa |
| pyrazon (pre-, early post-) | Sugarbeets |
| silvex | Fence lines, rights-of-ways |
| dichloroprop (post-) | Brush, Release of Evergreens |
| MCPB (early post-) | peas |
| dicamba (pre-) | flax and rape |
| desmedipham (post-) | Sugarbeets |
| prometryn (pre-) | Celery and Cotton |
| Phenmedipham (post-) | Sugarbeets |
| acifluorofen (Blazer®) (post) | Soybeans |
| 2-chloro-N-[(4-methoxy-6-methyl-1,3,5-triazin-2-yl)aminocarbonyl]benzenesulfonamide | Wheat and flax |
| 1-methylethyl 2[[(4,6-dimethoxy-1,3,5-triazin-2-yl)aminocarbonyl]aminosulfonyl]]benzoate | Wheat and flax |
| 1-methylethyl 2[[(4-methoxy-6-methyl-1,3,5-triazin-2-yl)aminocarbonyl]aminosulfonyl]]benzoate | Wheat and flax |
| dinoseb (post-) | Soybeans |
| lenacil (pre-) | Sugarbeets |
| bromoxynil (post-) | Wheat and barley |
| fluometuron (pre-) | Cotton |

Example XI

Seeds of crabgrass (*Digitaria* spp.), barnyardgrass (*Echinochloa crusgalli*), wild oats (*Avena fatua*), *Cassia tora*, morningglory (*Ipomoea* spp.), cocklebur (*Xanthium* spp.), sorghum, corn, soybean, rice, wheat and nutsedge tubers were planted in a growth medium and treated preemergence with the chemicals dissolved in a nonphytotoxic solvent. At the same time, cotton having five leaves (including cotyledonary ones), bush beans with the third trifoliate leaf expanding, crabgrass with two leaves, barnyardgrass with two leaves, wild oats with one leaf, cassia with three leaves (including cotyledonary ones), morningglory with four leaves (including the cotyledonary ones), cocklebur with four leaves (including the cotyledonary ones), sorghum with three leaves, corn with three leaves, soybean with two cotyledonary leaves, rice with two leaves, wheat with one leaf, and nutsedge with three-five leaves were sprayed. Treated plants and controls were maintained in a greenhouse for sixteen days. All species were compared to controls and visually rated for response to treatment seven and sixteen days after treatment. The ratings are based on a numerical scale extending from 0 = no injury, to 10 = no kill. The accompanying descriptive symbols have the following meanings:

B = burn;
G = growth retardation;
C = chlorosis/necrosis;
U = unusual pigmentation;
E = emergence inhibition; and
H = formative effects.

The ratings for the compound tested by this procedure are shown in Table 7 for 7 days after treating.

Table 7

| | 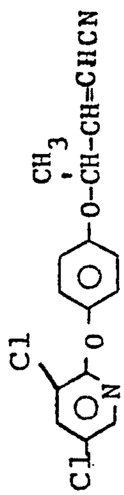 | 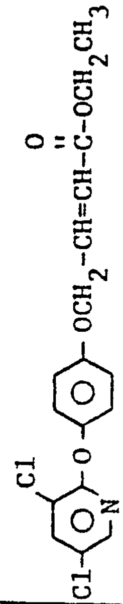 |
|---|---|---|
| kg/ha | 0.4 | 16 days 2 |
| POST-EMERGENCE | | |
| BUSHBEAN | 0 | 0 |
| COTTON | 0 | 1B |
| MORNINGGLORY | 0 | 0 |
| COCKLEBUR | 0 | 0 |
| CASSIA | 0 | - |
| NUTSEDGE | 0 | 0 |
| CRABGRASS | 10C | 9H |
| BARNYARDGRASS | 10C | 3C,9H |
| WILD OATS | 7C | 2S,5H |
| WHEAT | 5C | 0 |
| CORN | 5C | 2S,7H |
| SOYBEAN | 0 | 0 |
| RICE | 3C,9G | 1C |
| SORGHUM | 9C | 2S,7H |
| PRE-EMERGENCE | | |
| MORNINGGLORY | 0 | 0 |
| COCKLEBUR | 0 | 0 |
| CASSIA | 0 | 0 |
| NUTSEDGE | 0 | 0 |
| CRABGRASS | 3C,8G | 8H |
| BARNYARDGRASS | 10C | 9H |
| WILD OATS | 2C,8G | 5G |
| WHEAT | 4G | 0 |
| CORN | 5C,9G | 2C,9H |
| SOYBEAN | 0 | 0 |
| RICE | 3C,9G | 2C,7G |
| SORGHUM | 5C,9G | 9G |

Table 7 (continued)

| | 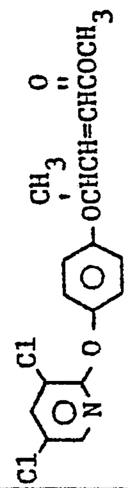 | | 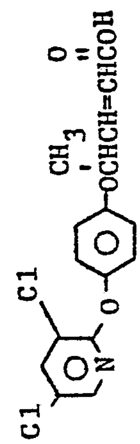 | |
|---|---|---|---|---|
| kg/ha | 2 | 0.4 | 2 | 0.4 |
| POST-EMERGENCE | | | | |
| BUSHBEAN | 0 | 0 | 0 | 0 |
| COTTON | 1B | 0 | 0 | 0 |
| MORNINGGLORY | 0 | 0 | 0 | 0 |
| COCKLEBUR | 1B | 0 | 0 | 0 |
| CASSIA | 1B | 0 | 0 | 0 |
| NUTSEDGE | – | 0 | 0 | 0 |
| CRABGRASS | 10C | 10C | 10C | 10C |
| BARNYARDGRASS | 10C | 10C | 10C | 10C |
| WILD OATS | 10C | 10C | 10C | 8C |
| WHEAT | 4G | 1C | 8C | 5C |
| CORN | 8C | 10C | 10C | 10C |
| SOYBEAN | 10C | 10C | 2C | 3C |
| RICE | 9C | 9G | 9C | 9C |
| SORGHUM | 10C | 10C | 10C | 10C |
| PRE-EMERGENCE | | | | |
| MORNINGGLORY | 0 | 0 | 3G | 0 |
| COCKLEBUR | 0 | 0 | 3G | 0 |
| CASSIA | 0 | 0 | 3G | 0 |
| NUTSEDGE | 0 | 0 | 8G | 0 |
| CRABGRASS | 10E | 10E | 10E | 10E |
| BARNYARDGRASS | 10E | 10E | 10C | 10E |
| WILD OATS | 10E | 10E | 10C | 8C |
| WHEAT | 7G | 6G | 6G | 4G |
| CORN | 9G | 9G | 10E | 9G |
| SOYBEAN | 0 | 0 | 0 | 0 |
| RICE | 10E | 10E | 10E | 10E |
| SORGHUM | 10E | 9G | 10E | 10E |

Table 7 (continued)

| | 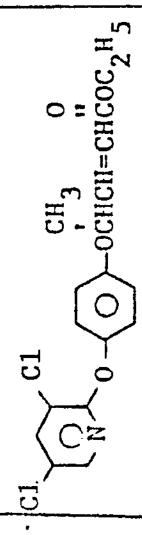 | 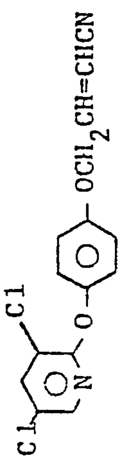 | |
|---|---|---|---|
| kg/ha | 0.4 | 0.4 | |
| POST-EMERGENCE | | | |
| BUSHBEAN | 0 | 1C,1H | |
| COTTON | 0 | 1C,5G | |
| MORNINGGLORY | 0 | 0 | |
| COCKLEBUR | 0 | 1C | |
| CASSIA | 1C | 0 | |
| NUTSEDGE | 0 | 0 | |
| CRABGRASS | 10C | 2H | |
| BARNYARDGRASS | 10C | 0 | |
| WILD OATS | 5C | 0 | |
| WHEAT | 0 | 0 | |
| CORN | 9C | 0 | |
| SOYBEAN | 1C | 0 | |
| RICE | 9C | 0 | |
| SORGHUM | 10C | 1C,6H | |
| PRE-EMERGENCE | | | |
| MORNINGGLORY | 0 | 0 | |
| COCKLEBUR | 0 | 0 | |
| CASSIA | 2C | 0 | |
| NUTSEDGE | 0 | 0 | |
| CRABGRASS | 10E | 2G | |
| BARNYARDGRASS | 10C | 3S,8H | |
| WILD OATS | 8G | 2S,4G | |
| WHEAT | 2G | 0 | |
| CORN | 9G | 4G | |
| SOYBEAN | 2H | 0 | |
| RICE | 10E | 0 | |
| SORGHUM | 9G | 2S,9H | |

Table 7 (continued)

| | 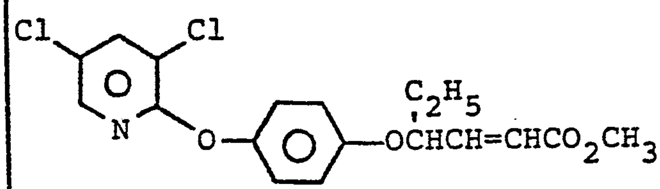 |
|---|---|
| kg/ha | 2 |
| POST-EMERGENCE | |
| BUSHBEAN | 0 |
| COTTON | 0 |
| MORNINGGLORY | 0 |
| COCKLEBUR | 0 |
| CASSIA | 0 |
| NUTSEDGE | 0 |
| CRABGRASS | 9C |
| BARNYARDGRASS | 7C |
| WILD OATS | 8G,3C |
| WHEAT | 0 |
| CORN | 3G |
| SOYBEAN | 0 |
| RICE | 0 |
| SORGHUM | 3G |
| PRE-EMERGENCE | |
| MORNINGGLORY | 0 |
| COCKLEBUR | 0 |
| CASSIA | 0 |
| NUTSEDGE | 0 |
| CRABGRASS | 9G |
| BARNYARDGRASS | 9C |
| WILD OATS | 7G |
| WHEAT | 6G |
| CORN | 6G |
| SOYBEAN | 4G |
| RICE | 10E |
| SORGHUM | 7G |

Claims:

1. A compound of the formula

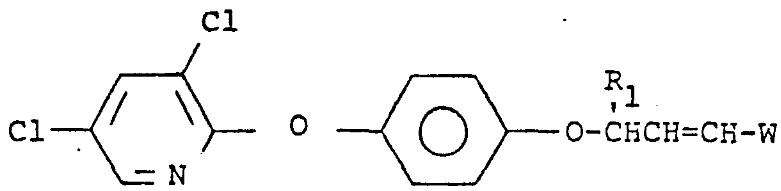

where

$R_1$ is H, $-CH_3$, $-CH_2CH_3$ or $-CH_2OCH_3$;
W is $COOR_2$, $CONR_3R_4$, $COSR_5$, COCl, CN or

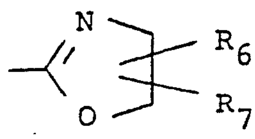 ;

$R_2$ is H, $C_1$-$C_4$ alkyl, $-CH_2CH_2OCH_3$, $-CH_2CH_2OCH_2CH_3$ or $-CH_2CH_2CH_2OCH_3$;
$R_3$ is H, $C_1$-$C_3$ alkyl or $-OCH_3$;
$R_4$ is H or $C_1$-$C_3$ alkyl;
$R_5$ is $C_1$-$C_4$ alkyl; and
$R_6$ and $R_7$ are independently H or $CH_3$;

and agriculturally suitable salts thereof.

2. A compound of claim 1 wherein $R_1$ is $CH_3$.

3. A compound of claim 1 or 2 wherein W is $COOR_2$ or CN.

4. A compound of claim 3 wherein $R_2$ is H, $CH_3$ or $C_2H_5$.

5. A compound of claim 4 wherein $R_2$ is H or $CH_3$.

6. Methyl 4-[4-(3,5-dichloropyridin-2-yloxy)-phenoxy]-2-pentenoate.

7. 4-[4-(3,4-Dichloropyridin-2-yloxy]phenoxy]-2-butenenitrile.

8. 4-[4-(3,5-Dichloropyridin-2-yloxy)phenoxy]-2-pentenenitrile.

9. 4-[4-(3,5-Dichloropyridin-2-yloxy)phenoxy]-2-pentenoic acid and agriculturally suitable salts thereof.

10. The D-enantiomers of the compounds of claims 1 to 9.

11. A composition for the control of undesirable vegetation comprising an effective amount of a herbicidal compound and at least one of (a) a surface active agent and (b) a solid or liquid diluent, characterised in that said herbicidal compound comprises a compound of any of claims 1 to 10.

12. A method for the control of undesirable vegetation by applying to the locus of such undesirable vegetation an effective amount of a herbicidal compound, characterised in that said herbicidal compound comprises a compound of any of claims 1 to 10.

13. A method for the selective control of grass weeds in broadleaf crops by applying to the locus of such grass weeds in effective amount of a herbicidal compound, characterised in that said herbicidal compound comprises a compound of any of claims 1 to 10.

14. A process for preparing a compound of Claim 1 where W is $CO_2R_2$ or CN which comprises contacting, in the presence of a base, the compounds

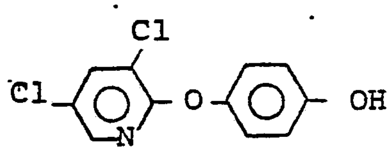 and 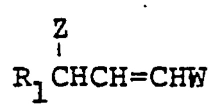

where $R_1$ and $R_2$ are as previously defined and where Z is Cl or Br.

15. The process of Claim 14 wherein the product is hydrolyzed with acid or base to yield a compound of Claim 1 where W = COOH.

16. A process for preparing a compound of Claim 1 where W is $-\overset{\overset{O}{\|}}{C}NR_3R_4$ which comprises contacting

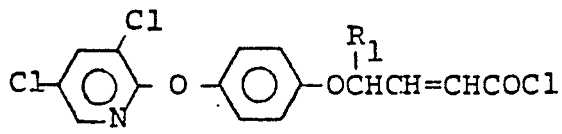

and

$HNR_3R_4$, where

$R_1$, $R_3$ and $R_4$ are as previously defined.

17. A process for preparing a compound of Claim 1 where W is $\overset{\overset{O}{\|}}{C}SR_5$ which comprises contacting the compounds

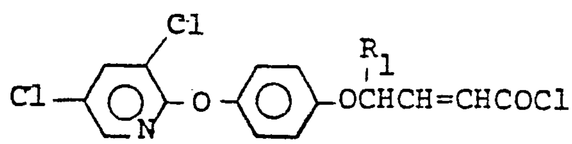

and

HSR5, where

$R_1$ and $R_5$ are as previously defined, in the presence of a base.

18. A process for preparing a compound of Claim 1 where W is COCl which comprises contacting a compound of the formula

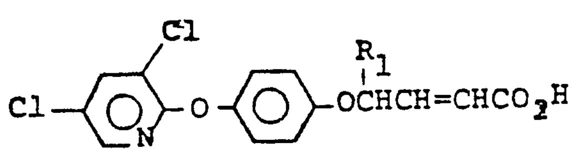

where $R_1$ is as previously defined, with oxalyl chloride or thionyl chloride.

19. A process for preparing a compound of claim 1 where W is

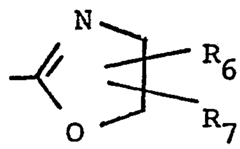

which comprises reacting the acid chloride defined in claim 16 with β-aminoethanol or a C-methyl or dimethyl-substituted derivative thereof and cyclizing the resulting amide.

0024907

European Patent Office

# EUROPEAN SEARCH REPORT

Application number
EP 80 30 2934

## DOCUMENTS CONSIDERED TO BE RELEVANT

| Category | Citation of document with indication, where appropriate, of relevant passages | Relevant to claim |
|---|---|---|
| PX | DE - A - 2 921 567 (KUMIAI CHEMICAL INDUSTRY CO. LTD) * Whole document * | 1-6,9, 11-13 |
| | -- | |
| | DE - A - 2 715 284 (ISHIHARA SANGYO KAISHA LTD) * Claims * | 1,11 |
| | -- | |
| | EP - A - 0 002 925 (IMPERIAL CHEMICAL INDUSTRIES) * Claims * | 1,11 |
| | -- | |
| | GB - A - 1 550 574 (CIBA-GEIGY) * Page 1 * | 1,11 |
| | -- | |
| D | BE - A - 875 880 (ISHIHARA SANGYO KAISHA LTD) * Claims * | 1,11 |
| | -- | |
| D | DE - A - 2 812 571 (ISHIHARA SANGYO KAISHA LTD) | 1,11 |
| | ---- | |

CLASSIFICATION OF THE APPLICATION (Int. Cl.3)

A 01 N 43/40
C 07 D 213/64
413/12

TECHNICAL FIELDS SEARCHED (Int. Cl.3)

C 07 D 213/64
213/63
413/12

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 07-11-1980 | VAN BIJLEN |